# EUROPEAN PATENT APPLICATION

(11) **EP 4 741 439 A1**
(43) Date of publication of application: **13.05.2026**
(21) Application number: 24839343.1
(22) Date of filing: 28.05.2024
(51) Int. Cl.: C08G 63/664, A61K 31/19, A61K 47/60, A61K 47/69, A61P 13/12, A61P 25/00, A61P 29/00, A61P 35/00, C08G 63/78, C08J 3/09, C08J 3/12

(54) **BLOCK COPOLYMER CONTAINING REPEATING UNIT OF POLY([R, S]BETA-HYDROXYBUTYRATE), NANOPARTICLES FORMED FROM SAID COPOLYMER, AND PRODUCTION AND USE OF THOSE**

(30) Priority: 07.07.2023 JP 2023112572
(71) Applicant: University of Tsukuba, Ibaraki 305-8577 (JP)
(72) Inventor: NAGASAKI, Yukio, Tsukuba-shi, Ibaraki 305-8577 (JP); DUC TRI, Bui, Tsukuba-shi, Ibaraki 305-8577 (JP)
(74) Representative: Kraus & Lederer PartGmbB
(86) International application number: PCT/JP2024/019499
(87) International publication number: WO 2025/013446

(57) **Abstract**

The present description discloses: a (ω-terminal modified) poly(ethylene glycol)-b-poly([R,S]-β-hydroxybutyrate) (PEG-b-PBHB) copolymer, nano-sized polymeric micelles or nanoparticles, which are formed from the copolymer and are capable of controlled release or sustained release of a low molecular weight β-hydroxybutyric acid in vivo; and production or preparation methods of these product, and a pharmaceutical use of the nanoparticles. Since the nanoparticles are capable of controlled release or sustained release of β-hydroxybutyric acid in vivo, physiological activity originally possessed by β-hydroxybutyric acid can be exhibited in vivo.

## Description

### Technical Field

The present invention relates to a block copolymer comprising a repeating unit of poly([R,S]β-hydroxybutyrate), and more particularly, to a poly(ethylene glycol)-b-poly([R,S]β-hydroxybutyrate) (PEG-b-PBHB) copolymer, and a production method and use thereof.

### Background Art

Since β-hydroxybutyric acid (BHB) which is one of body ketone bodies is involved in various signal transduction pathways (for example, energy metabolism, inflammatory response), its supplements provide benefits for treatment of many diseases. For example, Tajima T. et. al., Kidney international, 2019, 95 (5), 1120-1137 (hereinafter, it may be referred to as Non-Patent Document 1) report that injection of BHB reduces renal ischemia reperfusion injury if it has an effect on continuous delivery of BHB. However, BHB is a low molecular weight compound that is easily eliminated from the body after administration. Thus, it would be more advantageous to utilize physiological activity of BHB in therapeutic applications if it is possible to provide a controlled release (or sustained release) formulation of BHB.

One of strategies related to a current controlled release formulation is to produce an amphiphilic copolymer by PEGylation and then prepare nanoparticles from the copolymer for the purpose of increasing a molecular weight of a low molecular weight compound capable of releasing the low molecular weight compound via disintegration of nanoparticles and the amphiphilic copolymer. For example, WO 2020/166473 (hereinafter, it may be referred to as Patent Document 1) discloses that controlled-release nanoparticles (Nano^{L-DOPA}) of L-DOPA prepared from poly(ethylene glycol)-b-poly(L-DOPA) exhibit an advantageous therapeutic effect and reduce a detrimental effect by improving a pharmacokinetic profile of L-DOPA.

Indeed, a number of known research papers report on synthesis of diblock copolymers of poly(ethylene glycol) (PEG) and poly(β-hydroxybutyrate) (PBHB) with attempts at nanoparticle preparation (see, for example, Jeong, K. H. et. al., Macromolecular Research, 2008, 16, 418 - 423, Ravenelle, F. et. al., Biomacromolecules, 2003, 4 (3), 856 - 858).

In these studies, a diblock copolymer is synthesized by an esterification reaction between a PEG derivative and a natural poly([R]-β-hydroxybutyrate) hydrolysis product derived from commercially available bacteria and having a high molecular weight (MW 200,000 to 1,000,000 g/mol). Thus, flexibility for controlling the number of BHB units contained in the copolymer becomes poor. In addition, poly([R]-β-hydroxybutyrate) exhibits a high crystallization tendency and forms crystals rapidly, thus making self-assembly that occurs naturally in water difficult and requiring strict conditions for preparation of nanoparticles (CA 2430442 A1, hereinafter, may be referred to as Patent Document 2). For example, Patent Document 2 proposes preparation of nanoparticles by a time-consuming evaporation method from a copolymer as described above, in which a block copolymer is continuously stirred for a long time (that is, two weeks) in the form of an O/W emulsion to slowly evaporate an organic phase, thereby preparing target nanoparticles.

Therefore, if a means capable of sustained release or controlled release of β-hydroxybutyrate having an intended physiological activity can be obtained without the above-described problems or difficulties in the prior art, it would contribute to the progress of the art.

### Summary of Invention

### Technical Problem

From a pharmacological point of view, a small molecule BHB drug (sodium β-hydroxybutyrate) exhibits a very short half-life in vivo after administration (in particular, in an injection path), and therefore, its physiological activity is reduced. Therefore, an object of the present invention is to provide a means including a formulation capable of sustained release or controlled release of BHB having the physiological activity involved in various signal transduction pathways and the like.

From the viewpoint of material design, poly(β-hydroxybutyrate) (hereinafter, it may be referred to as "PBHB") is a hydrophobic polyester, and is not suitable for drug administration by an injection path because poly(β-hydroxybutyrate) causes aggregation in an aqueous environment. Accordingly, it is a further object of the present invention to provide a material that can be stably prepared in an aqueous environment and can be stably present.

### Solution to Problem

To the best of the present inventor's knowledge, it is undocumented that chemically synthesized PBHB, unlike natural or microorganism-derived or plant-derived poly([R]-β-hydroxybutyrate), exhibits the same physiological activity as natural BHB intended for pharmaceutical applications and the like.

On the other hand, since one carboxyl group and one hydroxyl group are simultaneously present in the structure of BHB, BHB can be bonded to each other via an ester bond to form a polymer. Actually, BHB in a polymerized form, that is, poly(β-hydroxybutyrate) (PBHB) can be produced at low cost by polymerizing β-butyrolactone, and is also easily chemically modified (for example, PEGylation, coating). Therefore, if chemically synthesized PBHB, particularly poly([R,S] β-hydroxybutyrate) can provide a material capable of exerting the same physiological activity as originally possessed by BHB in vivo, it would be one of possible approaches to solve the above problems.

In a diblock copolymer by an esterification reaction between a PEG derivative and a natural poly([R]-β-hydroxybutyrate) hydrolysis product disclosed in the prior art documents, in particular, the latter has a high tendency to be crystallized as described above, and thus preparation of a self-assembled composition thereof is originally in itself difficult. On the other hand, it has been found that, conveniently, when "R,S"-β-butyrolactone is subjected to ring-opening anionic polymerization in the presence of a PEG macroinitiator, and further a specific end group is introduced into a growth end (ω-end), it becomes easy to control the number of BHB units, and an ω-end modified diblock copolymer (poly(ethylene glycol)-b-poly([R,S]-β-hydroxybutyrate) (PEG-b-PBHB) copolymer (ω-end modified)) capable of suppressing a high crystallization tendency caused by the units can be provided. It has been confirmed that a plurality of molecules of the copolymer thus produced easily self-assemble or self-associate in water to form stable nanoparticles (or nano-sized polymeric micelles) (hereinafter, it may be referred to as "Nano^{BHB}"), and the nanoparticles perform sustained release of BHB in the presence of an esterase in vitro, and in vivo, increase a level of BHB in a specific organ, for example, exhibit an effect of protecting a model animal that has induced acute kidney injury.

Accordingly, the main subject matter or aspects disclosed herein may include:

### Aspect 1:

A block copolymer represented by Formula I: 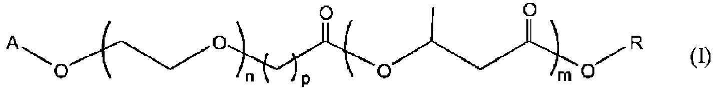 wherein
A represents a hydrogen atom, unsubstituted or substituted C₁-C₁₂ alkyl, unsubstituted or substituted C₁-C₁₂ alkoxy, or unsubstituted or substituted aryl, a substituent in a case of being substituted represents a C₁-C₄ alkyl, C₁-C₄ alkoxy, aryl, formyl, phenylamino, phenethylamino, or a group represented by Formula R¹R²CH- (wherein R¹ and R² are independently C₁-C₄ alkoxy or R¹ and R² together represent -OCH₂CH₂O-, -O(CH₂)₃O-, or -O(CH₂)₄O-),
R represents unsubstituted or substituted C₁₋₆ alkyl, C₁₋₄ alkylenecarbonyloxy-unsubstituted or substituted C₁₋₂₁ alkyl, C₁₋₄ alkylenecarbonyloxy-unsubstituted or substituted C₃₋₇ cycloalkyl, C₁₋₄ alkylenecarbonyloxy-unsubstituted or substituted aryl, C₁₋₄ alkylenecarbonyloxy-unsubstituted or substituted adamantyl, C₁₋₄ alkylene-unsubstituted or substituted aryl, C₁₋₄ alkylene-unsubstituted or substituted adamantyl, or C₁₋₄ alkylene-unsubstituted or substituted cholesterol residue, where a substituent in the case of being substituted represents C₁₋₄ alkyl, C₁₋₄ alkyloxy, or aryl,
m represents an integer of from 20 to 200,
n represents an integer of from 3 to 300,
p represents an integer of from 1 to 6, and a hydrogen atom of the alkylene with p attached is optionally substituted by one C₁₋₄ alkyl, and
in each β-hydroxylbutyrate unit in a repeating unit with m attached, equivalent amounts of an [R] type enantiomer and an [S] type enantiomer are present randomly from each other.

Aspect 2: A nano-sized polymeric micelle or nanoparticle comprising the block copolymer according to Aspect 1.

Aspect 3: A method for producing a block copolymer represented by Formula I according to the following Reaction Scheme 1, the production method comprising:
preparing a macroinitiator represented by Formula (2) by bringing a dissolved compound represented by Formula (1) into contact with an anionic polymerization catalyst that generates a metal cation represented by M⁺ in an organic solvent that does not adversely affect a reaction; and
reacting a [R,S]-β-lactone represented by Formula (3) with a reaction mixture obtained in the preparation, then stopping a polymerization reaction using a halogen substituent represented by Formula (4) and modifying an ω-end of a copolymer to produce a block copolymer represented by Formula I, and obtaining the produced block copolymer: 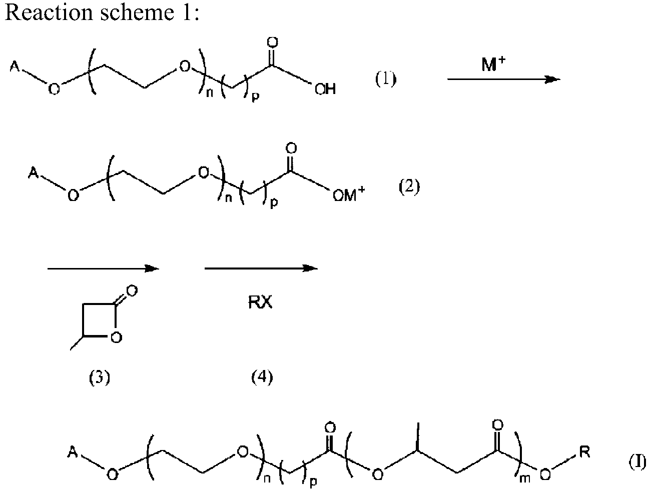 in abbreviations of a variable group or a variable part in each formula in the reaction scheme, A, R, m, n, and p having the same meaning as defined for the copolymer represented by Formula I according to Aspect 1, and X representing halogen.

Aspect 4: A method for preparing nano-sized polymeric micelles or nanoparticles of a block copolymer represented by Formula I, the method comprising:
forming nano-sized polymeric micelles or nanoparticles by dialysis of a mixture, prepared by adding water dropwise to a solution of a plurality of molecules of the block copolymer represented by Formula I and dissolved in a water-miscible organic solvent under stirring, against water through a dialysis membrane to prepare an aqueous solution comprising the nanoparticles; and
removing an aggregate from the aqueous solution.

Aspect 5: A pharmaceutical formulation containing the copolymer represented by Formula I according to Aspect 1 or the nano-sized polymeric micelle or nanoparticle according to Aspect 2 as an active ingredient, and further comprising a physiologically acceptable diluent or excipient.

Aspect 6: The pharmaceutical formulation according to Aspect 5 for use in the treatment of acute kidney disease, inflammatory diseases including ulcerative colitis, or brain diseases including depression.

Aspect 7: A method for preventing or treating one or two or more diseases of acute kidney disease, inflammatory disease, brain disease, and cancer, the method comprising administering an effective amount of the copolymer represented by Formula I according to Aspect 1, the nano-sized polymeric micelle according to Aspect 2, or a nano-sized polymeric micelle to a subject or patient in need of treatment.

### Aspect 8:

A block copolymer represented by Formula II or an alkali metal (Li, K, or Na) salt thereof: 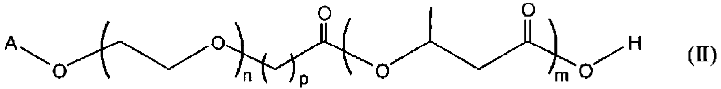
wherein A, R, m, n, and p have the same meaning as defined for the copolymer represented by Formula I according to Aspect 1, and
in each β-hydroxylbutyrate unit in a repeating unit with m attached, equivalent amounts of an [R] type enantiomer and an [S] type enantiomer are present randomly from each other.

### Advantageous Effects of Invention

According to the present invention, there is provided a diblock copolymer forming nano-sized polymeric micelles or nanoparticles, which is suitable for the purpose of use as a medicament by being in a form that is stable and sufficiently dispersed in water, and is capable of sustained release or controlled release of β-hydroxybutyrate having physiological activity involved in various signal transduction pathways and the like in vitro or in vivo. Therefore, according to the present invention, for example, a pharmaceutical formulation that can be used for prevention or treatment of acute kidney disease and the like is provided.

### Brief Description of Drawings

FIG. 1 is a conceptual diagram concerning formation of nano-sized polymeric micelles or nanoparticles provided in the present invention.
FIG. 2 shows a ¹H-NMR spectrum (A) and a ¹C-NMR spectrum of an ω-end modified copolymer produced in Production Example 1.
FIG. 3 is a view showing a measurement result of gel filtration chromatography of the ω-end modified copolymer.
FIG. 4 is a graph of data on (2) Polymerization (polymerization kinetics) in Production Example 1.
FIG. 5 is a graph of data on (3) Copolymers having different degrees of polymerization in Production Example 1.
FIG. 6 shows measurement results of a hydrodynamic size of Nano^{BHB} prepared in Production Example 2 by a DLS method, wherein (A) represents (in water), (B) represents (in physiological saline), and (C) is a transmission electron microscope (TEM) image.
FIGS. 7a and 7b are views showing ¹H NMR spectra of an ω-end unmodified copolymer produced in Production Example 3 and a corresponding modified copolymer and measurement results of DSLs thereof in comparison with each other.
FIG. 8 is a graph showing release properties of BHB from Nano^{BHB} under an influence of esterase in vitro in Test Example 1.
FIG. 9 shows in vivo release properties of BHB from Nano^{BHB} after intraperitoneal injection in Test Example 2, a graph (A) showing a change in a level of BHB over time in plasma, and a graph (B) showing an area under a curve of BHB in plasma.
FIG. 10 shows in vivo release properties of BHB from Nano^{BHB} after subcutaneous injection in Test Example 3, a graph (A) showing a change in the level of BHB over time in plasma, a graph (B) showing a change in the level of BHB over time in liver, a graph (C) showing a change in the level of BHB over time in kidney, and a graph (D) showing an area under the curve of BHB in kidney.
FIG. 11 shows a data graph regarding a therapeutic effect by administration of subcutaneous injection of Nano^{BHB} on cisplatin-induced acute kidney injury (AKI) in Test Example 4 together with a scheme of animal experiment.
FIG. 12 shows a graph of data on the therapeutic effect by oral gavage of Nano^{BHB} on AKI in Test Example 5 together with the scheme of animal experiment.
FIG. 13 shows a graph of data on an anti-depressant effect of Nano^{BHB} by oral free drinking via a forced swim test in a healthy mouse in Test Example 6 together with the scheme of animal experiment.
FIG. 14 shows a graph of data on the effect of Nano^{BHB} on survival of an ulcerative colitis mouse in Test Example 7 together with the scheme of animal experiment.
FIG. 15 is a graph showing results of measuring cytotoxicity after incubation of Nano^{BHB} and BHB with BAEC cells for 24 hours in Toxicity Test Example 1.
FIG. 16 is a graph of data on confirmation of toxicity of Nano^{BHB} by subcutaneous injection of 2-1 in Toxicity Test Example 2.
FIG. 17 is a graph of data on confirmation of the toxicity of Nano^{BHB} by oral administration of 2-2 in Toxicity Test Example 2.

### Detailed Description of the Invention or Best Mode for Carrying Out the Invention

The terms used herein, in particular the technical terms, have meaning commonly used in the art, unless otherwise defined.

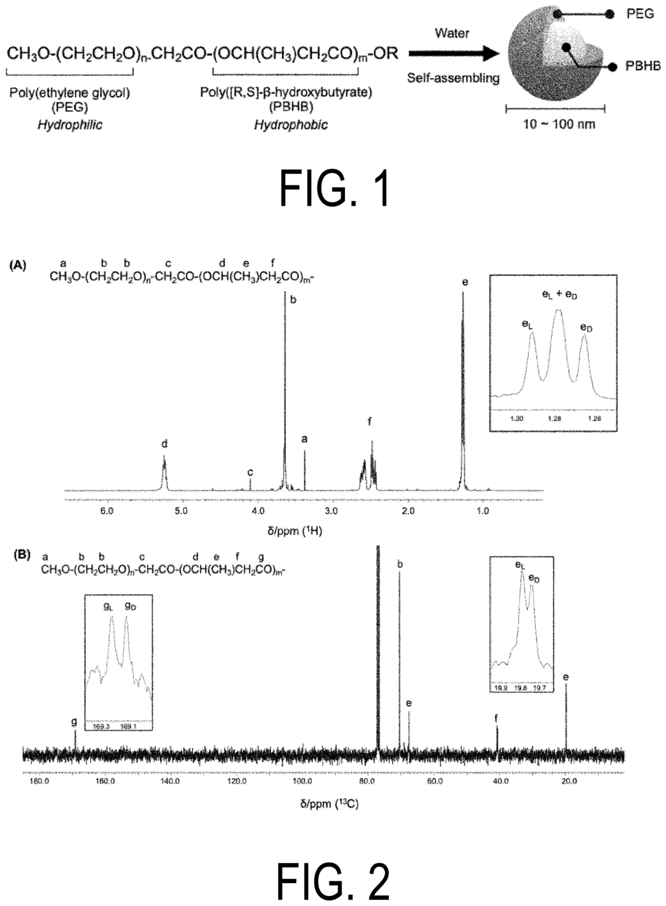

Nano-sized polymeric micelles and nanoparticles are used as interchangeable terms, and mean polymeric micelles or particles whose particle size distribution is within a nanosize range when particle size distribution measurement of the particles by dynamic light scattering (DLS) of the polymeric micelles is performed in an aqueous medium. Although not limited, from the viewpoint of stability, typical examples may include particles at from 5 nm to 500 nm, preferably from 5 nm to 100 nm, and more preferably from 7 nm to 80 nm. Hereinafter, for the purpose of simplifying the description, nano-sized polymeric micelles or nanoparticles are mainly referred to as nanoparticles. A conceptual diagram concerning the formation of such nanoparticles is shown in FIG. 1. Without being bound by theory, it is confirmed or understood that the nanoparticles are formed from a plurality of molecules of a block copolymer comprising a diblock of a poly(ethylene glycol) (PEG) segment and a poly([R,S]β-hydroxybutyrate) (PBHB) segment, particularly, a block copolymer in which an ω-end COOH group is protected by an appropriate hydrophobic group, and can exist as so-called core-shell type polymeric micelles having the (PBHB) segment as a core and the (PEG) segment as a shell in an aqueous environment (aqueous solution or in vivo).

The copolymer is as defined in the above-described Aspect 1, and may be collectively referred to as a poly(ethylene glycol)-b-poly([R,S]-β-hydroxybutyrate) copolymer (hereinafter, it may be referred to as "PEG-b-PBHB", ω-end modified). The described [R] and [S] are used according to R/S notation to distinguish absolute configuration of two isomers that may exist as enantiomers from each other. The recitation of [R,S] means that R-type enantiomer and S-type enantiomer may be present in equivalent amounts to each other, in other words, 50% of each other. When the notation [R, S] is used in the context of the present invention, the [R] form and the [S] form do not necessarily have to be present strictly in "equivalent amounts " or "50%" each with respect to each other, and for example, either isomer is present in excess to such an extent that the intended use is not adversely affected in [R,S]-β-hydroxybutyrate commercially available as a raw material for producing the copolymer, or in the case of the copolymer, even when the isomer unit of either one type is in slight excess depending on the production method, storage conditions, and the like thereof, either isomer depending on the raw material used and the like is understood to be within the technical scope of the present invention that can be tolerated as long as it conforms to the object of the present invention.

Regarding the variable group or the variable part that defines the copolymer:
A corresponds to a protecting group of an α-end OH group of a PEG segment similar to that of an α-end of the PEG segment disclosed in Patent Document 1, and R corresponds to a protecting group or a modifying group of ω-end COOH of PEG-b-PBHB. R is not limited as long as it is a hydrophobic group that acts to promote the formation of nanoparticles from enhancing hydrophobicity of a PBHB segment of PEG-b-PBHB, particularly a group containing a hydrocarbon group. However, from the viewpoint of facilitating the production of a PEG-b-PBHB copolymer (ω-end modified), which is one of the objects of the present invention, further facilitating the formation of nanoparticles from these copolymers, and stabilizing the formed nanoparticles in an aqueous medium, each group defined in Aspect 1 can be mentioned as a specific group.

Without limitation, according to a production method according to Reaction Scheme 1, R represents unsubstituted C₁₋₃ alkyl, C₁₋₂ alkylenecarbonyloxy-unsubstituted or substituted C₁₋₂₁ alkyl, C₁₋₂ alkylenecarbonyloxy-unsubstituted or substituted C₃₋₇ cycloalkyl, C₁₋₂ alkylenecarbonyloxy-unsubstituted or substituted aryl, C₁₋₂ alkylenecarbonyloxy-unsubstituted or substituted adamantyl, C₁₋₂ alkylene-unsubstituted or substituted aryl, C₁₋₂ alkylene-unsubstituted or substituted adamantyl, or C₁₋₂ alkylene-unsubstituted or substituted cholesterol residue, and here, the substituent in a case of being substituted is preferably a group representing C₁₋₄ alkyl, C₁₋₄ alkyloxy, or aryl, and when referred to as C₁₋₄ alkylenecarbonyloxy, C₁ alkylene (methylene) carbonyloxy is more preferable. In this case, R in RX in Reaction Scheme 1 can be an acetate residue of a hydrocarbon corresponding to the above-described group.

In m and n, in order for the nanoparticles to be easily and reliably formed from the block copolymer of Formula I in an aqueous environment and to be stably present as being of constant size, m can be an integer of from 20 to 200, preferably an integer of from 30 to 180, more preferably an integer of from 45 to 80, and n can be an integer of from 3 to 300, preferably an integer of from 10 to 100, more preferably an integer of from 20 to 100.

p is, for example, an integer varying corresponding to the fact that in the preparation of a macroinitiator of PEG, the integer oxidizes -CH₂CH₂OH, which is another end portion of an α-end-protected PEG, by a method known in the art to give -CH₂COOH, or generates an ether bond by reaction of OH of the end portion with a halo C₁₋₆ alkyl carboxylic acid ester, and then hydrolyzes the ester portion to convert it into a carboxyl group (-COOH). In consideration of ease of preparation, p is preferably an integer of from 1 to 3.

The alkyl or alkylene as each group or each portion described above can be linear or branched. Without being limited thereto, specific groups when referred to as C₁-C₁₂ alkyl may include those corresponding to methyl, ethyl, propyl, iso-propyl, butyl, sec-butyl, tert-butyl, pentyl, hexyl, heptyl, nonyl, undecyl, tridecyl, heptadecyl, nonadecyl, and the like. Preferred aryl is selected from C₁₋₆ alkyl. The alkylene described corresponds to the alkyl having the carbon atom described. The C₃₋₇ cycloalkyl can be cyclopropyl, cyclobutyl, cyclopentyl, cyclohexyl, or cycloheptyl, and aryl can be phenyl, biphenyl, or naphthyl.

Regarding production of the copolymer:
A method of producing the copolymer is not limited to any method as long as a copolymer having a structure represented by Formula I can be produced using a method known per se in the art. Advantageously, however, the copolymer can be produced based on Reaction Scheme 1 as described in Aspect 3. Here, the organic solvent that does not adversely affect the reaction can be an aprotic solvent, particularly a dipolar aprotic solvent, and can be, for example, tetrahydrofuran (THF), dioxane, glyme, and dimethylformamide (DMF). An anionic polymerization catalyst that produces a metal cation represented by M⁺ can be an alkali metal aromatic adduct such as K-naphthalene, Na-naphthalene, Na-biphenyl, K-biphenyl, or K-benzophenone.

Referring to Reaction Scheme 1, a slightly excess molar amount of the above alkali metal aromatic adduct is added to a PEG derivative represented by Formula (1) to prepare a PEG initiator represented by Formula (2), and [R,S]-β-lactone is subjected to ring-opening polymerization via the initiator thus prepared, as necessary, in the presence of a crown ether, for example, 18-crown-6-ether. These reactions are preferably performed under a nitrogen atmosphere. The amount of β-lactone used can be selected according to the number of m of a repeating unit of [R,S]-β-hydroxybutyrate of the intended copolymer represented by Formula I. Specifically, in the ring-opening polymerization reaction, the amount of β-lactone may be determined in consideration of the fact that a growth end grows in a living manner, or the polymerization reaction may be appropriately stopped using a halogen substituent described later to control the molecular weight or the number of m of the repeating unit. On the other hand, the number of n of the repeating unit of ethylene glycol of the copolymer represented by Formula I can be controlled by appropriately selecting the number of corresponding units of ethylene glycol in the PEG derivative to be provided. The polymerization reaction can be usually carried out for 1 day or from 2 days to several days. The halogen substituent used for the reaction termination or the protection of the ω-end, indicated as RX, can be chosen from the groups defined for R, described above, as R, and can be, as X, a halogen atom, particularly a bromine atom or an iodine atom. The copolymer (ω-end modified) represented by Formula I thus obtained can be separated and obtained from the reaction mixture by precipitation in a poor or non-solvent (for example, a mixture of isopropyl alcohol and n-hexane) for the copolymer. By adding a compound capable of providing a proton before the reaction termination with such a halogen substituent and protection of the ω-end, for example, water, to a polymerization reaction mixture, the copolymer represented by Formula II in which the ω-end -COOH is in a free state can be provided. Such a copolymer is also not described in any prior art document as long as the present inventor knows, and can also be said to be a precursor of the copolymer represented by Formula I in the present application.

The preparation or production of nanoparticles from the copolymer (ω-end modified) represented by Formula I can be performed according to the preparation method described in Aspect 4. In the preparation, as long as the object of the present invention is not violated, the copolymer represented by Formula II may be contained as an ω-end unprotected product, for example, up to 25 mol% of a pre-copolymer.

In the preparation method, as a water-miscible organic solvent, a good solvent that can easily dissolve the copolymer (ω-end modified) of Formula I, for example, DMF can be used. While the solution thus dissolved is stirred as vigorously as possible, a dose of water substantially equal to the solution is added dropwise to prepare a mixture. The mixture is dialyzed against a 20-40 times dose of water of an untreated solution (if necessary, the water is changed twice a day) through an RC dialysis membrane of an appropriate cut-off molecular weight (for example, 3.5 kDa) for from 1 to 3 days. In the thus obtained nanoparticle-containing solution from which the organic solvent and an unreacted raw material have been removed, an aggregate coexisting in the solution is removed using a filter having a suitable pore size as necessary. Thus, the nanoparticles (Nano^{BHB}) present in an aqueous solution or aqueous suspension usually have a dynamic mechanical average diameter of 7 or from 10 nm to 800 nm as measured by DLS, and among these, more stable nanoparticles may have a dynamic mechanical average diameter of from 7 nm to 80 nm.

The nanoparticles can be separated by separation means such as centrifugation or filtration treatment, can be stored as a dry composition by freeze-drying, and can be reconstituted in an aqueous medium as necessary. Thus, Nano^{BHB} can be provided as a formulation for oral administration or a formulation for parenteral administration (intraperitoneal administration, intravenous injection, subcutaneous injection, etc.), which contains a physiologically acceptable diluent or excipient, as necessary. Such a diluent may be sterile water, physiological saline, a solution containing a physiologically acceptable buffer, or the like, and may further contain, as an additive, for example, sorbitol, dextrin, glucose, mannitol, an amino acid (for example, glycine, isoleucine, valine, methionine, glutamic acid, or the like), or the like.

Although the technical scope of the present invention is not limited by theory, when Nano^{BHB} is injected into a living body, NanoBHB accumulates in a region injured by an enhanced vascular permeability/retention (EPR) effect, and then nanoparticle disintegration and sustained release of BHB by enzymatic hydrolysis of PBHB occur. Consequently, it is understood that the use of Nano^{BHB} improves pharmacokinetics of a small molecule BHB and enhances the physiological activity. As confirmed in Test Example 4 described later, poly(ethylene glycol)-b-poly([R,S]-β-hydroxybutyrate) shows a protective effect against acute kidney injury using an experimental animal.

Examples of the dosage form of the pharmaceutical formulation include liquid preparations (including solutions for internal use, suspensions, emulsions, and syrups), solid preparations (including tablets, pills, sublingual tablets, capsules, drops, and troches), granules, powders, and powdered drugs. If necessary, the solid preparation can be further formulated into a dosage form coated with a coating known in the art, for example, a sugar-coated tablet, a gelatin-coated tablet, an enteric coated tablet, a film-coated tablet, a double tablet or a multilayer tablet. The shape and size of each dosage form may be within the range of dosage forms known in the art, and additives such as solubilizers, pH adjusters, carriers, excipients, diluents, binders, disintegrators, lubricants, emulsifiers, extenders, colorants, flavoring agents, sweeteners, stabilizers, and preservatives can be contained.

The dose and administration frequency of Nano^{BHB} as an active ingredient in these formulations are applied in an optimal concentration range depending on the age, sex, condition, degree of disease, and the like of the patient; however, an amount in terms of Nano^{BHB} to be administered per day is preferably from about 10 µmg to about 1 g, more preferably from 0.1 mg to 100 mg per Kg of body weight, and this amount may be divided into about 1 to 3 times. However, since the pharmaceutical formulation according to an embodiment of the present invention has high bioavailability, the effect can be expected even if it is administered once a day. With respect to the specific dose, administration method, and administration frequency, those skilled in the art can determine an optimum method through animal experiments or experimental administration to humans with reference to various literatures and the like.

### Examples

### Production Example 1: Poly(ethylene glycol)-b-poly([R,S]-β-hydroxybutyrate) (PEG-b-PBHB) copolymer (ω-end modified): Production of CH₃O-(CH₂CH₂O)ₙ-CH₂CO-(OCH(CH₃)CH₂CO)ₘOCH₂COOCH₂CH₃

### Process 1. Production of PEG-macroinitiator: CH₃O-(CH₂CH₂O)ₙ-CH₂COOH

### (a) Synthesis of CH₃O-(CH₂CH₂O)ₙ-CH₂COOC₂H₃

Under a stream of N2, butyllithium 1.6 M-hexane (BuLi, 10 mL, 16 mmol) was added to a solution of CH₃O-(CH₂CH₂O)ₙ-H (mPEG-OH, MW = 2000, 20 g, 10 mmol) dissolved in tetrahydrofuran (THF, 50 mL), and subsequently ethyl bromoacetate (3.6 mL, 30 mmol) was added thereto. After 1 day of reaction, the product was precipitated in 2-propanol (cold IPA) stored at -30°C and collected by centrifugation (9000 rpm, 30 minutes). The product was dried under reduced pressure (yield: 18 g).

### (b) Synthesis of CH₃O-(CH₂CH₂O)ₙ-CH₂COOH

MiliQ water (50 mL) was added to CH₃O-(CH₂CH₂O)ₙ-CH₂COOC₂H₃ (18 g, 9 mmol) at room temperature, and subsequently NaOH1.5M (10 mL, 15 mmol) was added thereto.

After stirring for 1 day, pH of a reaction system was adjusted to from 1 to 2 using hydrochloric acid. The product was then extracted with dichloromethane (DCM, three times, 60 mL each). A DCM layer was concentrated on a rotary evaporator under reduced pressure and precipitated in cold IPA. The product was collected by centrifugation (9000 rpm, 5 minutes) and dried under reduced pressure (yield: 16 g).

### Process 2.

### (1) Polymerization (typical example)

### Method:

Under a stream of N₂, CH₃O-(CH₂CH₂O)ₙ-CH₂COOH (2 g, 1 mmol) provided as described above was dissolved in THF (15 mL), and subsequently, naphthalene potassium 0.9 M/hexane (1.5 mL, 1.35 mmol) was added. Then, 18-crown-6 ether 1M/THF (2 mL, 2 mmol) was introduced into the mixture obtained above, followed by addition of [R,S]-β-butyrolactone (available from Tokyo Chemical Industry Co., Ltd., 4 mL, 49 mmol). After the reaction for 2 days, ethyl bromoacetate (4 mL, 33 mmol) was added to stop the reaction. The reaction mixture was precipitated in IPA/n-hexane (20/80), and the product was collected by centrifugation (9000 rpm, 10 minutes) and dried under reduced pressure.

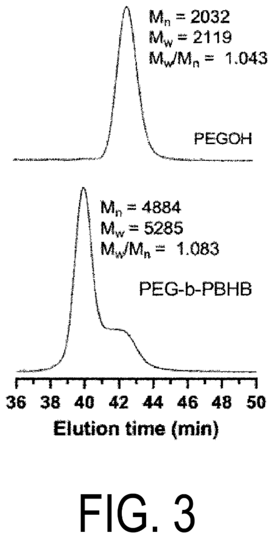

The yield of product: CH₃O-(CH₂CH₂O)ₙ-CH₂CO-(OCH(CH₃)CH₂CO)ₘ was 5.6 g. A ¹H-NMR spectrum and a ¹³C-NMR spectrum of the product are shown in FIGS. 2(A) and 2(B), respectively. A molecular weight M converted from the result of the ¹H-NMR spectrum is 6,300, and contains 52 BHB units. In an enlarged spectral diagram, an S-unit (e_{S}, g_{S}) and an R-unit (e_{R}, g_{R}) can be observed, and it can be confirmed that the configuration of the obtained copolymer is a poly(ethylene glycol)-b-poly([R,S]-β-hydroxybutyrate) (PEG-b-PBHB) copolymer (ω-end modified). The measurement results of gel filtration chromatography (GPC) of the copolymer are shown in FIG. 3. It can be seen from FIG. 3 that Mn = 4884 and Mw/Mn = 1.083.

### (2) Polymerization (kinetics of polymerization)

### Method:

According to the treatment described in the above (1) Polymerization, [R,S]-β-butyrolactone was added, and then 0.5 mL of tetralin was added as a standard of the NMR spectrum. After the chosen time point (for example, 30 minutes, 18 hours, 21 hours, 40.5 hours), two drops of the reaction mixture were collected and frozen in liquid nitrogen. Thereafter, a sample was mixed with CDCl₃ for NMR measurement and THF for GPC measurement.

### Results:

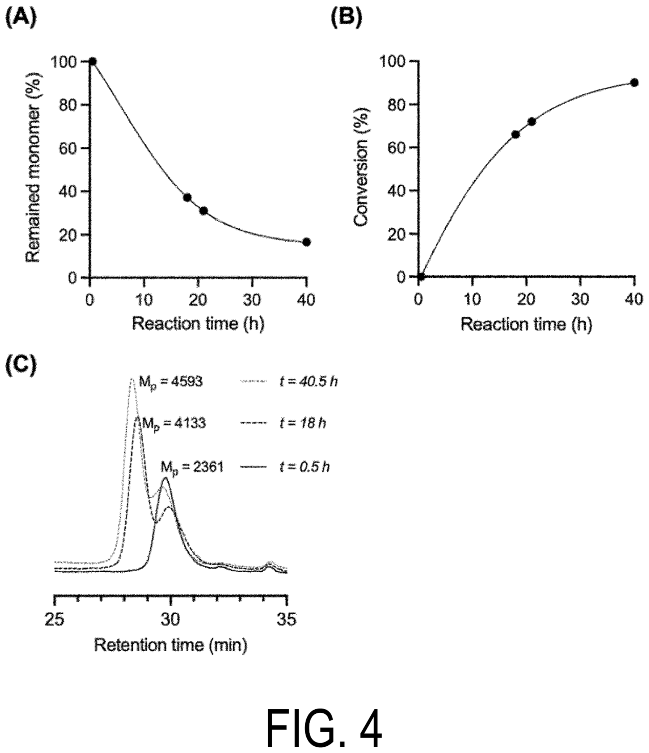

A residual amount of [R,S]-butyrolactone was relatively measured from ¹H-NMR, and normalized using tetralin as a reference peak. The results of an experiment concerning the kinetics of the polymerization are shown in FIG. 4. Here, (A) is a graphical representation of a change in residual monomer over time, (B) is a graphical representation of the result of monitoring a conversion of monomer to polymer by ¹H-NMR in CDCl₃, and (C) is a graphical representation of the result of GPC measurement of the product in THF. 80% or more of [R,S]-butyrolactone, respectively, was consumed after 40.5 hours, and an increase in poly (BHB) signal was also observed with reaction time, with a first peak shifting to a high molecular weight region with increasing the reaction time, suggesting polymerization on a PEG macromer.

### (3) Copolymer having different degrees of polymerization

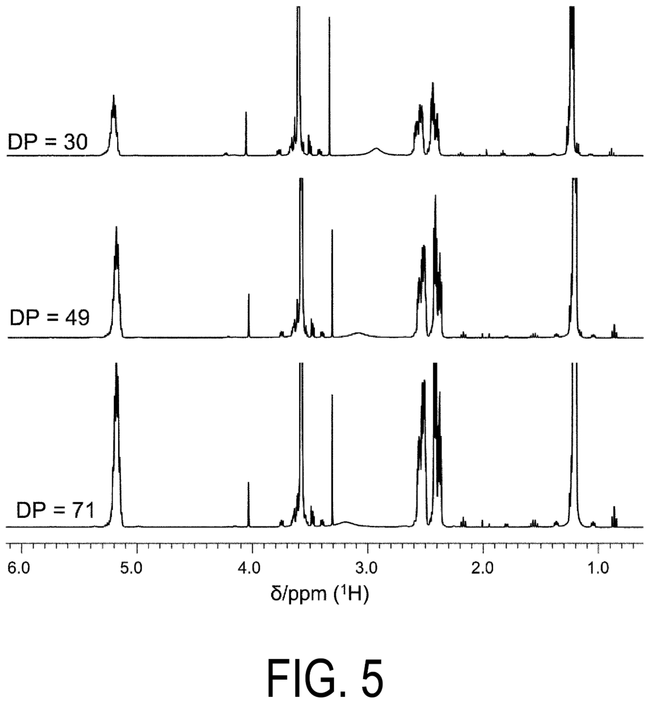

According to the method described in the above (1) Polymerization, following the introduction of 18-crown-6-ether, different amounts of [R,S]-β-butyrolactone (that is, 2 mL, 4 mL, and 6 mL) were added. After the reaction for 2 days, acetic acid was added to stop the polymerization reaction. The product was collected by dialysis against methanol, dimethylformamide (DMF) and then MiliQ water in a 3.5 kDa RC dialysis membrane and subsequently dried by lyophilization under reduced pressure. The ¹H-NMR spectrum of each obtained product is shown in FIG. 5. Degrees of polymerization (DP) of a PBHB segment in each product converted from these spectra are P1 (DP = 30), P2 (DP = 49), and P3 (DP = 71), respectively. For the PEG segment in each product, see (1) above.

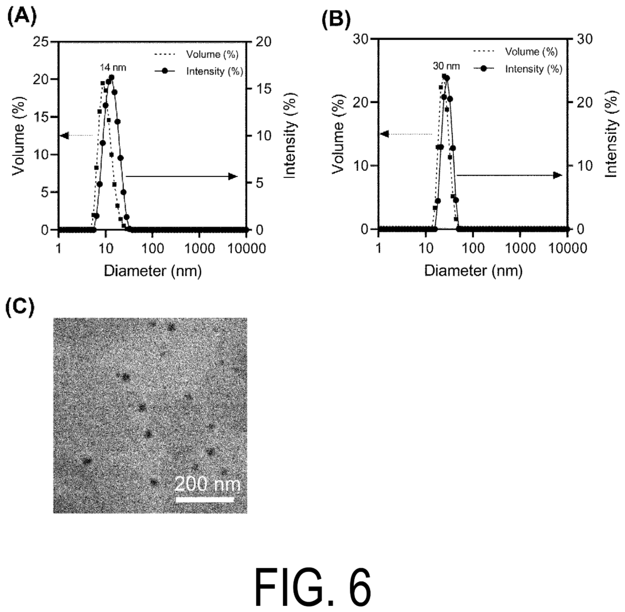

Production Example 2: Preparation of Nano^{BHB} Nanoparticles
(1) The poly(ethylene glycol)-b-poly([R,S]-β-hydroxybutyrate) (PEG-b-PBHB) copolymer (ω-end modified) (5.6 g) produced as described in Process 2 of Production Example 1 was dissolved in 40 mL of DMF. 40 mL of MiliQ water was then added dropwise to the solution under vigorous stirring. The obtained whole mixture was dialyzed against 2 L of MiliQ water through a 3.5 kDa RC dialysis membrane for 3 days (water was changed twice per day). In the last process, the nanoparticle solution was filtered through a 0.2 µm-pore size filter to remove an aggregate and then sterilized. For injection purposes, nanoparticles in physiological saline were prepared by mixing the nanoparticle solution with 9% NaCl (9:1). All of the nanoparticles were stored at 4°C for further use.
   The measurement results of the product by dynamic light scattering (DLS) and the measurement results of a transmission electron microscope are shown in FIG. 6. Here, (A) is the measurement result of a Nano^{BHB} solution (32 mg/mL, d = 14 nm, V = 170 mL, yield: 97%) in the MiliQ water, (B) is the measurement result of a Nano^{BHB} solution (28.8 mg/mL, d = 30 nm) in physiological saline, and (C) is the measurement result of a transmission electron microscope.
(2) PEG-b-PBHB copolymers (hereinafter, referred to as a P1 copolymer, a P2 copolymer, and a P3 copolymer, respectively) each having P1 (DP = 30), P2 (DP = 49), and P3 (DP = 71) were provided as the PBHB segments obtained in the production of (3) Copolymers having different degrees of polymerization in Process 2 in Production Example 1, and self-assembly of each copolymer, removal of an aggregate, and sterilization were performed according to the method described in (1) of Production Example 2. The characteristics of the copolymers having different degrees of polymerization are summarized in the following Table 1.

**Table 1:**

| Copolymer | Reaction terminator | [I]₀/[M]₀ | DP^{a} | Mₙ^{a} | M_{w}^{b} | Dynamic mechanical average diameter ^{d} (nm) | PDI |
|---|---|---|---|---|---|---|---|
| P1 | Acetic acid | 1 : 33 | 30 | 4580 | 4217 | 768.0 ± 367.2 | 0.27 |
| P2 | Acetic acid | 1 : 66 | 49 | 6200 | 5285 | 14.7 ± 6.7 | 0.23 |
| P3 | Acetic acid | 1 : 99 | 71 | 8106 | 5862 | 14.6 ± 6.7 | 0.38 |
| | | | | | | 98.2 ± 44.7 | |

| | | | | | | | |
|---|---|---|---|---|---|---|---|
| a: converted value from ¹H-NMR measurement result b: value obtained from GPC measurement result d: value obtained from DLS measurement result | | | | | | | |

### Production Example 3: Poly(ethylene glycol)-b-poly([R,S]-β-hydroxybutyrate) (PEG-b-PBHB) copolymer (ω-end unmodified)

### Production of CH₃O-(CH₂CH₂O)ₙ-CH₂CO-(OCH(CH₃)CH₂CO)ₘOH

In the polymerization in Process 2 in Production Example 1, the treatments described in Processes 1 and 2 in Production Example 1 were repeated except that water or acetic acid was added to stop the reaction instead of "ethyl bromoacetate (4 mL, 33 mmol) was added to stop the reaction". Thus, an ω-end unmodified copolymer and the like were produced.

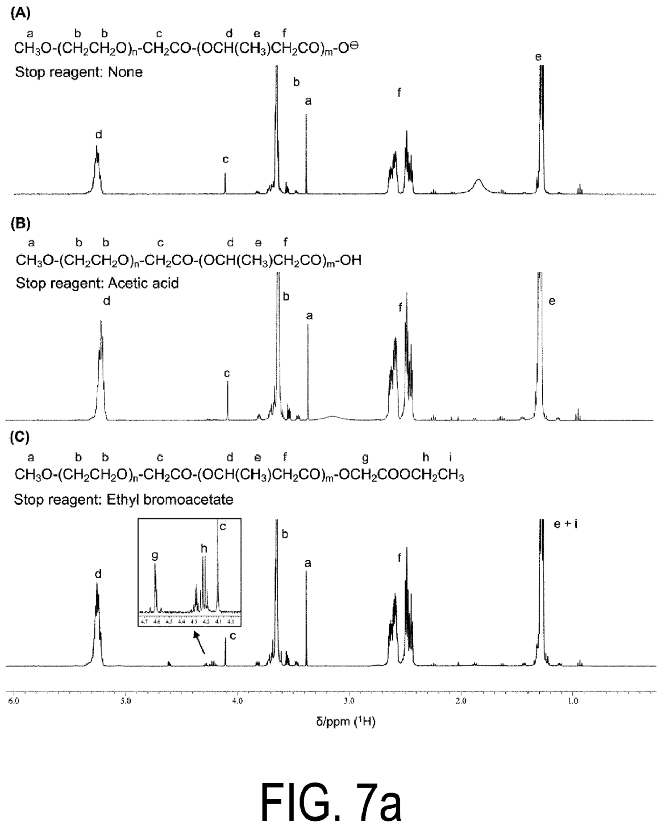

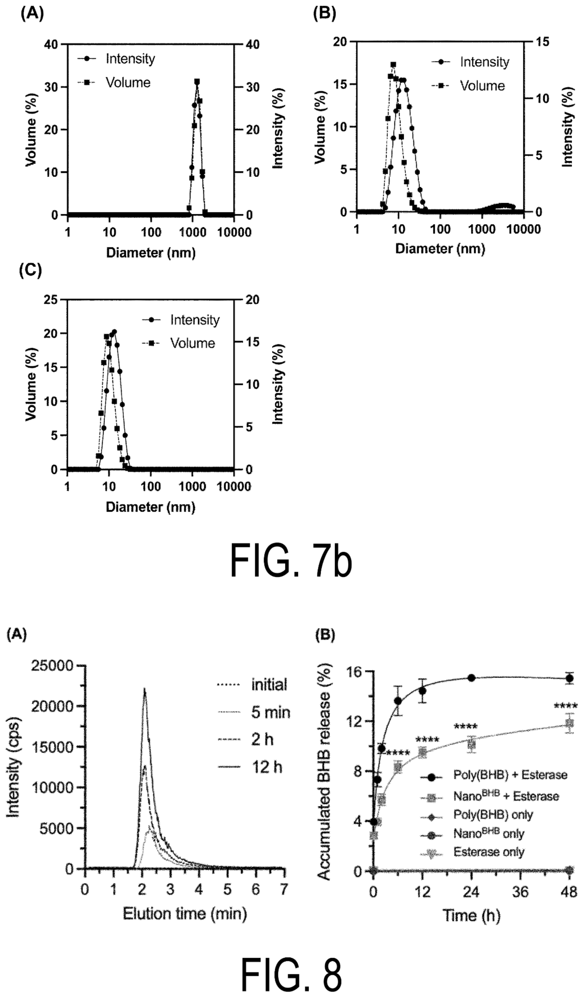

For the purpose of examining the effect of modifying the ω-end group, a copolymer obtained when the reaction was stopped using water and the end group was unmodified (COO⁻), a copolymer obtained when the reaction was stopped using acetic acid and the end group was (COOH), and a modified (the end group: COOCH₂CH₃) copolymer (see Production Example 1) were each made into nanoparticles according to the preparation method of nanoparticles of Production Example 2. The ¹H-NMR spectrum of each copolymer described above and the measurement result of DLS for each nanoparticle are shown in comparison with FIGS. 7a and 7b, respectively. From the figure, it is understood that the copolymer in which the end group is unmodified (COO⁻) and DP is 45 forms large particles. Such properties are less desirable in the formation of a hydrophobic core. Although the copolymer having the end group (COOH) and a DP of 49 forms small nanoparticles, a certain degree of aggregation is observed even after filtration. This is likely due to some degree of end group (COOH) being protonated in water. It is understood that the copolymer in which the end group is modified with an ester group and DP is 52 increases the hydrophobicity and forms small and stable nanoparticles.

### Test Example 1: Release of BHB from Nano^{BHB} in vitro by enzyme

(1) Experiment: 19 mg/mL (1 mL, BHB content of 0.15 mmol) of Nano^{BHB} was dispersed in 3 mL of an aqueous solution of pig liver esterase (0.33 mg/mL) and incubated at 37°C. After the chosen time point (that is, 5 minutes, 2 hours, 6 hours, 12 hours, 24 hours, 48 hours), 100 µL of the reaction mixture was collected (n = 3) and immediately frozen in liquid nitrogen. After sample collection at t = 48 hours, 1M (1 mL, 1 mmol) of KOH was added and incubated for 4 days to allow complete hydrolysis, at which time t = ∞ (infinity) was assumed. An accumulated release amount of BHB was measured with an LC-MS/MS system. A similar experiment was also performed with PBHB (19 mg/mL). Accumulated BHB was calculated as BHB (%) = [BHB]ₜ × 100%/[BHB]t = ∞ (infinity).
(2) Results:
   The results of the above experiment are shown in FIG. 8. (A) is a BHB-detected LC-MS/MS chromatograph of a mixture of Nano^{BHB} and esterase over time, and (B) is a result of quantitative analysis of a BHB level in an incubation solution over time (**** p < 0.0001 vs. Nano^{BHB} + esterase group).

The following can be seen from the figure:
Nano^{BHB} can release free BHB under the influence of esterase in vitro, providing an evidence for a strategy of the present inventors for using Nano^{BHB} as a source of BHB (see (A)).

In Nano^{BHB}, BHB release is slower and more stable compared to a homogeneous PBHB polymer (see (B)).

### Test Example 2: In vivo release of BHB by intraperitoneal injection of Nano^{BHB}

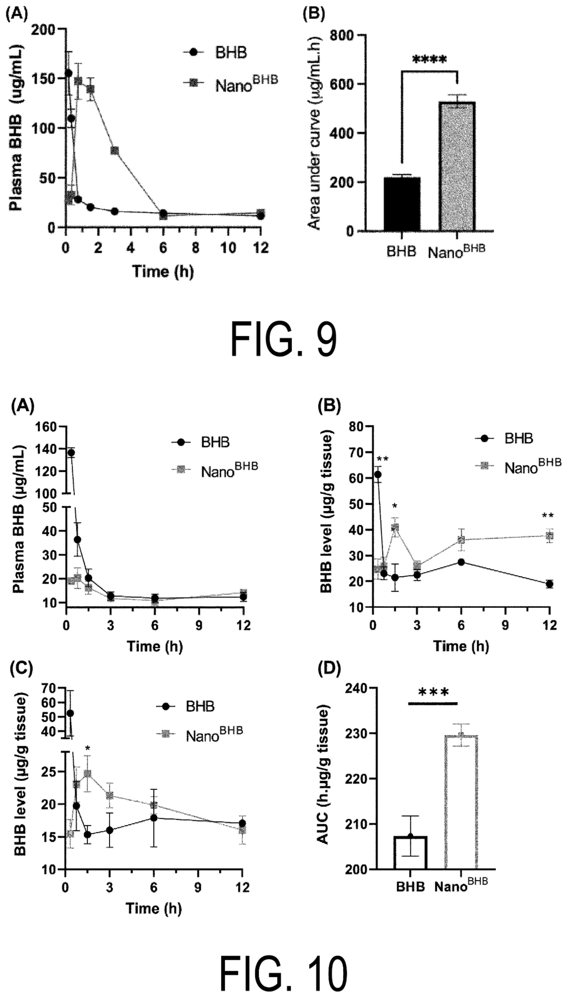

(1) Experiment:
   Nano^{BHB} or sodium β-hydroxybutyrate was administered to an ICR male mouse (6 weeks old) by intraperitoneal injection at a dose of 345 mg in terms of BHB/kg. After the chosen time point (that is, 20 minutes, 45 minutes, 1.5 hours, 3 hours, 6 hours, 12 hours), the mouse was sacrificed, and the plasma was collected. A plasma sample was immediately frozen in liquid nitrogen and stored at -80°C for further analysis. The BHB level in the plasma sample was measured with an LC-MS/MS system.
(2) The results of the above experiment are shown in FIG. 9. In the figure, (A) is the level of BHB in plasma after the intraperitoneal injection, and (B) is an area under a curve showing the BHB level in plasma at from 0 to 12 hours. From the figure, the intraperitoneal injection of Nano^{BHB} significantly increases the level of BHB in plasma and shows a better pharmacokinetic profile than normal exogenous BHB.

### Test Example 3: In vivo release of BHB by subcutaneous injection of Nano^{BHB}

(1) Experiment:
   Nano^{BHB} or sodium β-hydroxybutyrate was administered to an ICR male mouse (6 weeks old) by subcutaneous injection at a dose of 345 mg in terms of BHB/kg. After the chosen time point (that is, 20 minutes, 45 minutes, 1.5 hours, 3 hours, 6 hours, 12 hours), the mouse was sacrificed, and the plasma, kidney, and liver were collected. Plasma and tissue samples were immediately frozen in liquid nitrogen and stored at -80°C for further analysis. The BHB levels in the plasma, the kidney and the liver were measured with the LC-MS/MS system.
(2) The results of the above experiment are shown in FIG. 10. In the figure, (A) is the BHB level in plasma, (B) is the BHB level in liver tissue, (C) is the BHB level in kidney tissue, and (D) represents an area under the BHB level curve in kidney tissue, each data after subcutaneous injection.

From the figure, subcutaneous injection of Nano^{BHB} did not significantly increase the level of BHB in the blood. However, it is found that the BHB levels in the kidney and liver are increased.

### Test Example 4: Protective effect of Nano^{BHB} on acute kidney injury by subcutaneous injection

### (1) Experiment:

C57BL/6J male mice (8 weeks old) were divided into four groups for use in different treatments. Acute kidney injury was induced by intraperitoneal injection of cisplatin (20 mg/kg). A mouse was pretreated with subcutaneous injection of physiological saline (n = 11) or sodium β-hydroxybutyrate (at a dose in terms of BHB/kg of about 250 mg, n = 1) or Nano^{BHB} (at a dose in terms of BHB/kg of about 250 mg, n = 10) 6 hours prior to exposure to cisplatin. The healthy group (n = 8) was injected with physiological saline instead of cisplatin. 72 hours after the cisplatin challenge, the mouse was sacrificed, and blood plasma and kidney were collected. Renal function was assessed by measuring the levels of blood urea nitrogen (BUN), creatinine (CRE), aspartate transaminase (AST).

### (2) Results:

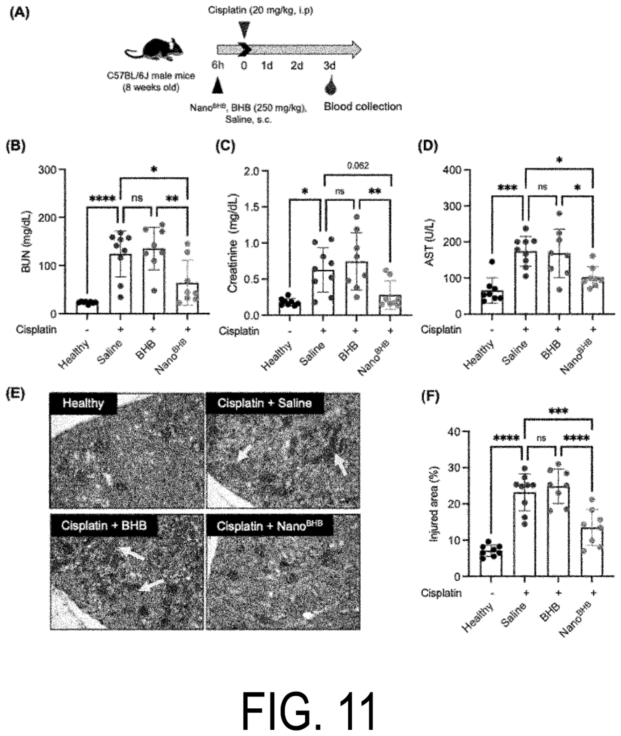

The therapeutic effect by administration of subcutaneous injection of Nano^{BHB} on cisplatin-induced acute kidney injury (AKI) is shown in FIG. 11 together with a scheme of animal experiment. In the figure, (A) represents a scheme of animal experiment, (B) is a graphical representation of data on blood urea nitrogen in plasma of an AKI mouse 3 days after injection of cisplatin, (C) is a graphical representation of data on creatinine, (D) is a graphical representation of data on aspartate transaminase, (E) is a presentation image of periodic acid Schiff (PAS) staining at 20 fold magnification of kidney tissue, and (F) is a graph summarizing data of the result of quantitative measurement of a damaged region of the kidney from a stained image. (Data are expressed as mean + SD, n = 8 - 9, *p < 0.05, **p < 0.01, ***p < 0.001, ****p < 0.0001, one-way ANOVA).

These figures confirm that cisplatin has induced significant impairment in the kidney, as confirmed by increases in renal function biomarkers (BUN, CRE) and AST. Although no decrease in renal function is observed by subcutaneous injection of physiological saline and sodium β-hydroxybutyrate, it is understood that administration of Nano^{BHB} exhibits a remarkable protective effect against cisplatin-induced acute kidney injury. It is also shown that Nano^{BHB} acts more advantageously than low molecular weight BHB.

### Test Example 5: Therapeutic effect of Nano^{BHB} on acute kidney injury by forced oral administration (oral gavage)

### (1) Experiment:

C57BL/6J male mice (8 weeks old) were divided into four groups and treated differently. Acute kidney injury was induced by intraperitoneal administration of cisplatin (20 mg/kg). Physiological saline (n = 10), sodium β-hydroxybutyrate (at a dose in terms of BHB/kg of about 250 mg, n = 10) or Nano^{BHB} (at a dose in terms of BHB/kg of about 250 mg, n = 10) was administered via oral gavage every 2 days (day -3, -1). The healthy group (n = 10) was injected with physiological saline instead of cisplatin. 72 hours after the cisplatin challenge, the mouse was sacrificed, and plasma and kidney were collected. Kidney function was assessed by measuring the values of blood urea nitrogen (BUN), creatinine (CRE), and aspartate transaminase (AST). In all cisplatin injected groups, a mouse with a plasma creatinine level of less than 0.2 mg/dL was considered cisplatin resistant and excluded from data analysis.

### (2) Results:

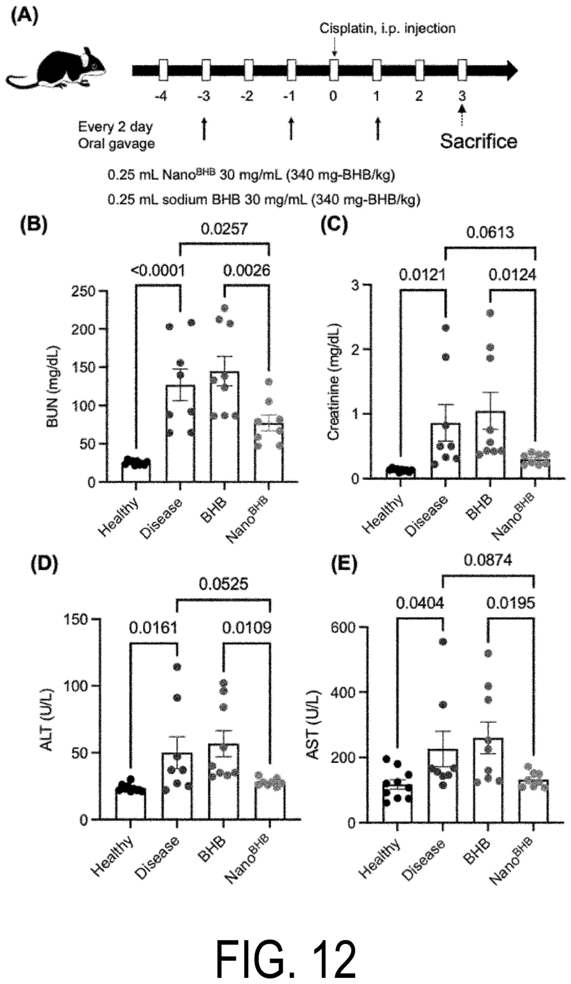

The therapeutic effect by oral gavage of Nano^{BHB} on AKI is shown in FIG. 12 together with the scheme of animal experiment. In the figure, (A) is a scheme of animal experiment, (B) is a measured value of blood urea nitrogen in plasma of an AKI mouse 3 days after injection of cisplatin, (C) is a measured value of creatinine, (D) is a measured value of alanine transaminase, and (E) is a measured value of aspartate transaminase. Data are expressed as mean ± SD, n = 8-10, based on one-way ANOVA.

From each figure, it can be understood from low values of BUN, CRE, ALT, and AST that Nano^{BHB} exhibits a better therapeutic effect than BHB sodium by oral gavage every two days. This suggests a persistence of the effect by oral gavage of Nano^{BHB}, and it is understood that a frequency of administration of a drug can be reduced by using Nano^{BHB} as compared with the low molecular weight BHB in the prior art.

### Test Example 6: Anti-depressant effect of Nano^{BHB} by free drinking

### (1) Experiment:

Any of water, Nano^{BHB} (in terms of BHB: 20 mM), and sodium β-hydroxybutyrate (in terms of BHB: 20 mM) was administered to a male C57BL/6 mouse (9 weeks old) via a free drinking bottle for 3 days. The forced swim test, described in (Nature 1977,266:730-732), was performed with some modifications. Here, each mouse was placed in a 2 L plastic beaker filled with water at 25°C at a water level at which the mouse's tail did not touch the bottom, behavior for 6 minutes was recorded with a video recorder, and the time of swimming behavior (mobility) and floating behavior (immobility) was measured.

### (2) Results:

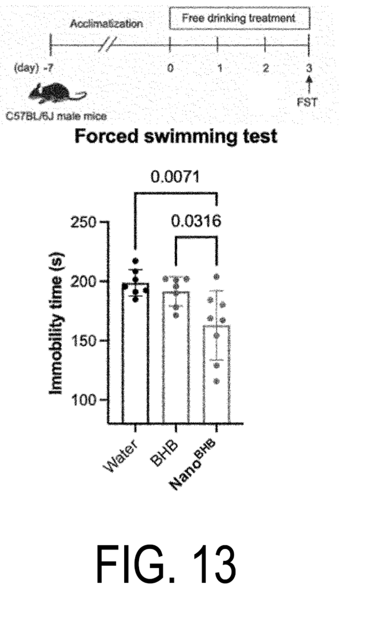

An anti-depressant effect of Nano^{BHB} by oral free drinking via the forced swim test in a healthy mouse is shown in FIG. 13 with the scheme of animal experiment. Data in the figure are based on mean ± SD, n = 7-8, and one-way ANOVA. From the figure, a depression-like behavior during the forced swim test was reduced by free drinking of Nano^{BHB}, and an anti-depression-like effect was observed. In addition, it is understood that Nano^{BHB} exhibits a statistically significantly excellent antidepressant-like action as compared with a case where NanoBHB is administered at a BHB concentration of 20 mM, which is comparable to that of sodium β-hydroxybutyrate in the prior art.

### Test Example 7: Effect of Nano^{BHB} on survival of ulcerative colitis mouse

### (1) Experiment:

Male C57BL/6J mice (7 weeks old) were fed ad libitum with water (healthy mice) or dextran sulfate sodium 2.5% (DSS). DSS drinking mice were administered with any of water, sodium β-hydroxybutyrate, and Nano^{BHB} (dose: 70 mg per day in terms of BHB/kg) by oral gavage at 0.2 mL/mouse every two days. The survival rate of the mouse was observed for 16 days.

| Group (n = 12) | Ad libitum intake | Oral gavage (every 2 days) |
|---|---|---|
| Healthy | Tap water | MQ water |
| Colitis | Dextran sulfate sodium 2.5% | MQ water |
| BHB | Dextran sulfate sodium 2.5% | BHB sodium |
| | | BHB/kg · day conversion 70 mg |
| Nano ^{BHB} | Dextran sulfate sodium 2.5% | Nano^{BHB} |
| | | BHB/kg · day conversion 70 mg |

### (2) Results:

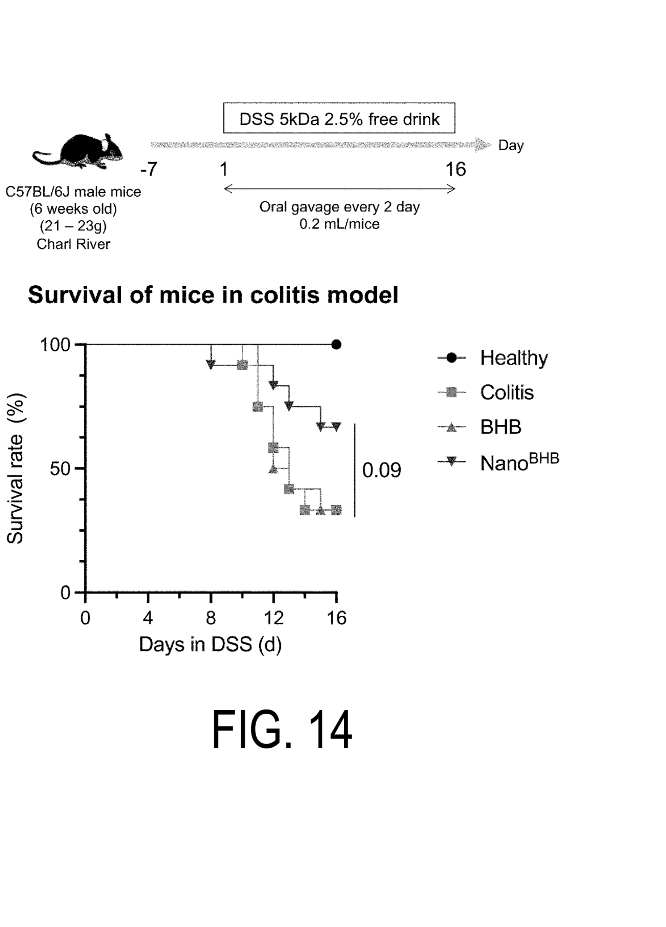

The effect of oral gavage of Nano^{BHB} on the survival of a DSS-induced ulcerative colitis mouse is shown in FIG. 14 along with the scheme of animal experiment.

From the figure, there is a tendency that a survival rate of DSS-induced colitis mouse is improved by administration of Nano^{BHB}.

### Toxicity Test Example 1: Cytotoxicity of Nano^{BHB}

### (1) Experiment:

Bovine aortic endothelial cells (BAEC) (available from RIKEN (RIKEN, Tsukuba, Japan)) were pre-seeded on a 96 well (well) plate and incubated with either Nano^{BHB} or BHB at different concentrations with a BHB content of from 0 to 25.4 mM in a medium. After 24 hours, the medium was discarded, followed by addition of 100 µL of medium containing 10 µL of WST agent and incubation for 4 hours. In a final process, cell viability was measured based on a ratio of optical signals of a subject well and a water treatment group well at a wavelength of 450 nm in each well.

### (2) Results:

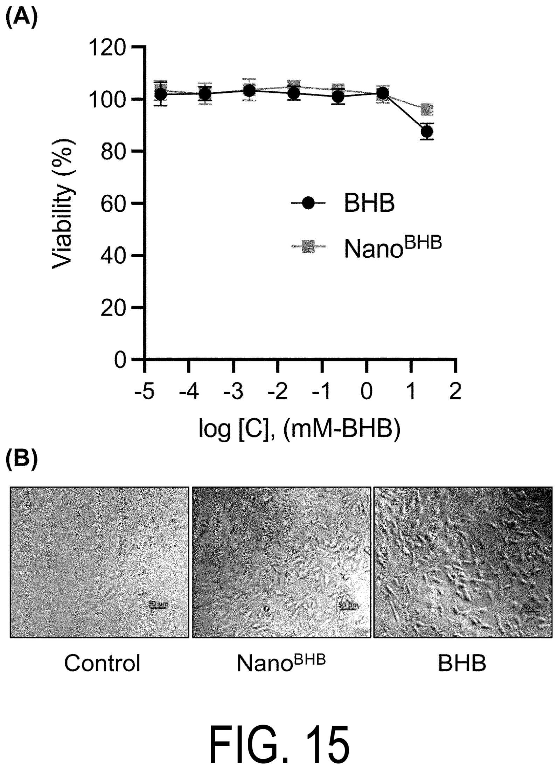

The results are shown in FIG. 15. In the figure, (A) represents the survival rate (mean ± SD, n = 4) of bovine aortic endothelial cells (BAEC), and (B) is a representative photomicrograph image (scale bar 50 µm) of BAEC cells after culturing for 24 hours. From the figure, it is understood that both Nano^{BHB} and BHB do not affect the survival rate and morphology of the BAEC cells and show low toxicity in vitro to normal cells.

### Toxicity Test Example 2: In vivo toxicity of Nano^{BHB} in healthy mouse

### 2-1 Confirmation of toxicity of Nano^{BHB} by subcutaneous injection:

### (1) Experiment:

C57BL/6J male mice (8 weeks old) were divided into 2 groups with different treatments. Physiological saline (n = 5) or Nano^{BHB} (about 250 mg in terms of BHB/kg, n = 5) was injected subcutaneously. After 75 hours, the mouse was sacrificed, and plasma and kidney were collected. The kidney function was assessed by measuring the levels of blood urea nitrogen (BUN), creatinine (CRE), and aspartate transaminase (AST).

### (2) Results

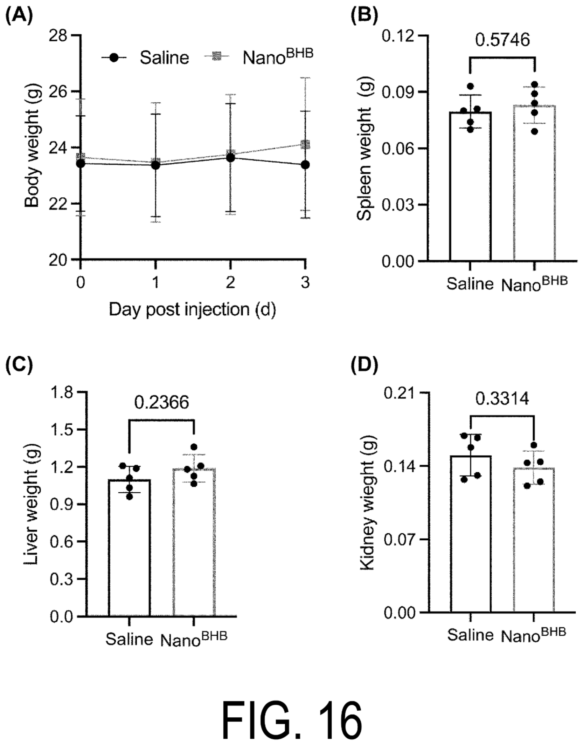

The results are shown in FIG. 16 and Table 2. The figure shows results of in vivo toxicity experiment by one-shot subcutaneous injection (s. c.) in a mouse, wherein (A) represents a change in body weight of a test animal and (B) represents the weight of spleen, liver and kidney 3 days after injection.

Table 2 summarizes data of a complete blood count and results of plasma biochemical analysis (data is expressed as mean + SD, n = 5) of a blood sample taken 3 days after injection.

**Table 2: Complete blood count and plasma biochemical analysis**

| | Physiological saline | Nano^{BHB} | p value |
|---|---|---|---|
| White blood cells (10²/µL) | 35.6 ± 5.4 | 35.6 ± 9.2 | 1.00 |
| Red blood cells (10⁴/µL) | 854.4 ± 38.1 | 826.6 ± 23.9 | 0.20 |
| Hemoglobin (g/dL) | 13.1 ± 0.5 | 12.9 ± 0.6 | 0.65 |
| Platelet (10⁴/µL) | 82.1 ± 1.5 | 78.5 ± 12.3 | 0.54 |
| ALT (U/L) | 23.2 ± 3.3 | 25 ± 3.6 | 0.40 |
| AST (U/L) | 53.4 ± 9.4 | 58.2 ± 11.7 | 0.50 |
| BUN (mg/dL) | 29.7 ± 2.6 | 30.9 ± 2.1 | 0.45 |
| CRE (mg/dL) | 0.17 ± 0.01 | 0.22 ± 0.06 | 0.18 |

| | | | |
|---|---|---|---|
| ALT = alanine transaminase | | | |

From FIG. 16 and Table 2, no significant toxicity by s. c. administration of Nano^{BHB} was observed, and no statistically significant difference in body weight and organ weight (spleen, kidney, liver) was observed between the groups. There is no difference in kidney and liver disorder biomarkers compared to a physiological saline administration group as well, suggesting that s. c. injection of Nano^{BHB} is safe in this model.

### 2-2 Confirmation of toxicity of Nano^{BHB} by oral administration:

### (1) Experiment:

C57BL/6J male mice (8 weeks old) were divided into two groups and treated differently. Physiological saline (n = 5) or Nano^{BHB} (about 250 mg - BHB/kg, n = 5) was orally administered every two days (day 0, 2, 4). The mouse was sacrificed on the sixth day, and plasma and kidney were collected. The kidney function was assessed by measuring the levels of blood urea nitrogen (BUN), creatinine (CRE), and aspartate transaminase (AST).

### (2) Results

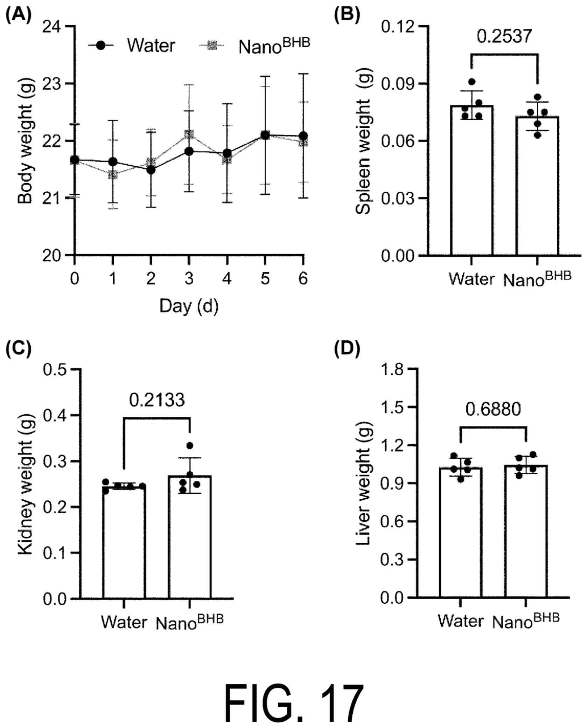

The results are shown in FIG. 17 and Table 3. The figure shows data representing the toxicity when Nano^{BHB} is orally administered to a mouse every two days, wherein (A) represents a change in body weight of a test animal, and (B) shows weights of spleen, liver, and pancreas 6 days after the first administration. Table 3 summarizes the data of the complete blood count and the results of plasma biochemical analysis (data is expressed as mean ± SD, n = 5) of a blood sample taken 6 days after the first dose.

**Table 3: Complete blood count and plasma biochemical analysis**

| | Water | Nano^{BHB} | p value |
|---|---|---|---|
| White blood cells (10²/µL) | 57.2 ± 17.3 | 63.2 ± 14.8 | 0.572 |
| Red blood cells (10⁴/µL) | 858.4 ± 29.6 | 869.2 ± 12 | 0.47 |
| Hemoglobin (g/dL) | 13.7 ± 0.4 | 13.9 ± 0.3 | 0.48 |
| Platelet (10⁴/µL) | 35.1 ± 10.8 | 39.2 ± 16.6 | 0.66 |
| ALT (U/L) | 24.6 ± 3.4 | 26 ± 4.2 | 0.579 |
| AST (U/L) | 114.6 ± 40.4 | 67.2 ± 14.7 | 0.04 |
| BUN (mg/dL) | 26.8 ± 1 | 27 ± 1.4 | 0.73 |
| CRE (mg/dL) | 0.14 ± 0.02 | 0.11 ± 0.02 | 0.02 |

From FIG. 17 and Table 3, the oral administration of Nano^{BHB} does not affect body weight, organ weight, and overall blood components, and safety by oral administration of Nano^{BHB} is suggested. In addition, an action of decreasing a functional disorder biomarker (AST, CRE, etc.) is observed, and it is understood that it is also effective in healthy mice.

## Claims

1. A block copolymer represented by Formula I: 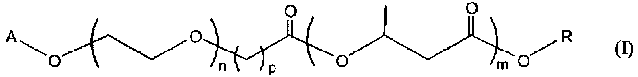 wherein
A represents a hydrogen atom, unsubstituted or substituted C₁-C₁₂ alkyl, unsubstituted or substituted C₁-C₁₂ alkoxy, or unsubstituted or substituted aryl, a substituent in a case of being substituted represents a C₁-C₄ alkyl, C₁-C₄ alkoxy, aryl, formyl, phenylamino, phenethylamino, or a group represented by Formula R¹R²CH- (wherein R¹ and R² are independently C₁-C₄ alkoxy or R¹ and R² together represent -OCH₂CH₂O-, -O(CH₂)₃O-, or -O(CH₂)₄O-),
R represents unsubstituted or substituted C₁₋₆ alkyl, C₁₋₄ alkylenecarbonyloxy-unsubstituted or substituted C₁₋₂₁ alkyl, C₁₋₄ alkylenecarbonyloxy-unsubstituted or substituted C₃₋₇ cycloalkyl, C₁₋₄ alkylenecarbonyloxy-unsubstituted or substituted aryl, C₁₋₄ alkylenecarbonyloxy-unsubstituted or substituted adamantyl, C₁₋₄ alkylene-unsubstituted or substituted aryl, C₁₋₄ alkylene-unsubstituted or substituted adamantyl, or C₁₋₄ alkylene-unsubstituted or substituted cholesterol residue, where a substituent in the case of being substituted represents C₁₋₄ alkyl, C₁₋₄ alkyloxy, or aryl,
m represents an integer of from 20 to 200,
n represents an integer of from 3 to 300,
p represents an integer of from 1 to 6, and a hydrogen atom of an alkylene with p attached is optionally substituted by one C₁₋₄ alkyl, and
in each β-hydroxylbutyrate unit in a repeating unit with m attached, equivalent amounts of an [R] type enantiomer and an [S] type enantiomer are present randomly from each other.

2. A nano-sized polymeric micelle or nanoparticle comprising the block copolymer according to claim 1.

3. A method for producing a block copolymer represented by Formula I according to Reaction Scheme 1, the production method comprising:
preparing a macroinitiator represented by Formula (2) by bringing a dissolved compound represented by Formula (1) into contact with an anionic polymerization catalyst that generates a metal cation represented by M⁺ in an organic solvent that does not adversely affect a reaction; and
reacting a [R,S]-β-lactone represented by Formula (3) with a reaction mixture obtained in the preparation, then stopping a polymerization reaction using a halogen substituent represented by Formula (4) and modifying an ω-end of a copolymer to produce a block copolymer represented by Formula I, and obtaining the produced block copolymer: 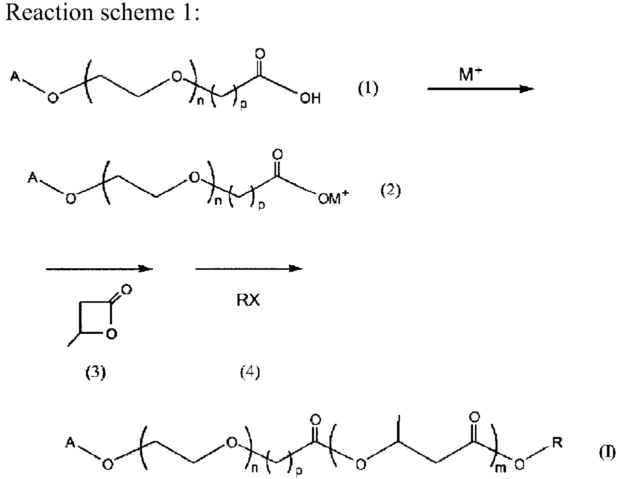
in abbreviations of a variable group or a variable part in each formula in the reaction scheme, A, R, m, n, and p having the same meaning as defined for the copolymer represented by Formula I according to claim 1, and X representing halogen.

4. A method for preparing nano-sized polymeric micelles or nanoparticles of a block copolymer represented by Formula I, the method comprising:
forming nano-sized polymeric micelles or nanoparticles by dialysis of a mixture, prepared by adding water dropwise to a solution of the block copolymer represented by Formula I according to claim 1 and dissolved in a water-miscible organic solvent under stirring, against water through a dialysis membrane to prepare an aqueous solution comprising the nanoparticles; and
removing an aggregate from the aqueous solution.

5. A pharmaceutical formulation comprising the copolymer represented by Formula I according to claim 1 or the nano-sized polymeric micelle or nanoparticle according to claim 2 as an active ingredient.

6. The pharmaceutical formulation according to claim 5, for use in a treatment of one or more diseases of acute kidney disease, inflammatory disease, brain disease, and cancer.

7. A method for preventing or treating one or more diseases of acute kidney disease, inflammatory disease, and brain disease, the method comprising administering an effective amount of the copolymer represented by Formula I according to claim 1 or the nano-sized polymeric micelle according to claim 2 to a subject or patient in need of treatment.

8. A block copolymer represented by Formula II or an alkali metal salt thereof: 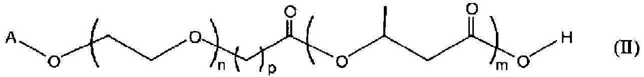
wherein A, R, m, n, and p have the same meaning as defined for the copolymer represented by Formula I according to claim 1, R represents a hydrogen atom, and
in each β-hydroxylbutyrate unit in a repeating unit with m attached, equivalent amounts of an [R] type enantiomer and an [S] type enantiomer are present randomly from each other.
